# EUROPEAN PATENT APPLICATION

(11) **EP 4 659 680 A1**
(43) Date of publication of application: **10.12.2025**
(21) Application number: 25176850.3
(22) Date of filing: 15.05.2025
(51) Int. Cl.: A61B 6/03, A61B 6/42, A61B 6/58, A61B 6/00

(54) **SYSTEMS AND METHODS FOR COMPUTED TOMOGRAPHY CALIBRATION**

(30) Priority: 04.06.2024 US 202418733758
(71) Applicant: GE Precision Healthcare LLC, Waukesha, WI 53188 (US)
(72) Inventor: FAN, Jiahua, Waukesha, 53188-1696 (US); LING, Jiayin, Waukesha, 53188-1696 (US); RAMANI, Sathish, Niskayuna, 12309-1027 (US); WU, Mingye, Niskayuna, 12309-1027 (US)
(74) Representative: AWA Sweden AB

(57) **Abstract**

Embodiments of a method for calibrating an imaging system are disclosed herein. In one example, the method includes generating a detector response prediction for one or more composition features of a non-uniform phantom, scanning the non-uniform phantom at a plurality of positions between an X-ray source and a detector of the imaging system, measuring an actual detector response at each position, generating a correction factor based on the detector response prediction and adjusting one or more calibration algorithms based on the correction factor.

## Description

### TECHNICAL FIELD

Embodiments of the subject matter disclosed herein relate to a phantom and more particularly, to a phantom used to calibrate a photon counting CT scanner.

### BACKGROUND

In computed tomography (CT) imaging systems, an electron beam generated by a cathode is directed towards a target within an X-ray source or X-ray tube. A fan-shaped or cone-shaped beam of X-rays produced by electrons colliding with the target is directed towards a subject, such as a patient. After being attenuated by the object, the X-rays impinge upon an array of X-ray detectors, generating an image. One example of a CT system is a photon counting CT (PCCT), where the X-ray detectors are photon-counting detectors, and photons are counted to provide spectral information. A calibration process may be performed periodically on the PCCT system to reduce the impact of charge sharing and detector crosstalk on images produced by the CT system. The calibration process may include performing a CT imaging procedure on an object, called a phantom, and generating a correction factor based on the resulting image of the phantom.

### SUMMARY

In one example, a method for a photon counting computed tomography (PCCT) system is provided. The method includes during a calibration of the PCCT system generating a detector response prediction for one or more known composition features of a non-uniform phantom, performing a calibration scan of the non-uniform phantom at a plurality of positions between an X-ray source and a detector of the PCCT system, measuring an actual detector response at each of the plurality of positions, generating a correction factor based on the detector response prediction and the actual detector response at each of the plurality of positions, and adjusting a calibration algorithm based on the correction factor.

The above advantages and other advantages and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings in which:
FIG. 1 shows a pictorial view of a photon counting computed tomography (PCCT) imaging system.
FIG. 2 shows a block schematic diagram of an example PCCT imaging system.
FIG. 3 shows a schematic diagram of an example phantom with example slots.
FIG. 4 shows a close-up view of the slots within an example phantom.
FIG. 5 shows a diagram of a phantom positioned within a PCCT system.
FIG. 6A shows a first cross sectional view of an elliptical phantom including beads positioned within a PCCT system.
FIG. 6B shows a second cross sectional view of the elliptical phantom including beads of FIG. 6A.
FIG. 6C shows a third cross sectional view of the elliptical phantom including beads of FIG. 6A.
FIG. 7 shows a flowchart describing a workflow for designing a phantom.
FIG. 8 shows a flowchart describing a calibration method for a PCCT system.
FIG. 9 shows a flowchart describing the calibration method for a PCCT system using a phantom with slots.
FIG. 10 shows a flowchart describing a method for applying calibration data to correct for detected error.
FIG. 11 shows a first embodiment of a rectangular prism phantom including beads.
FIG. 12 shows a first embodiment of a cylindrical phantom including beads.
FIG. 13 shows a second embodiment of a rectangular prism phantom including beads.
FIG. 14 shows a second embodiment of a cylindrical phantom including beads.

### DETAILED DESCRIPTION

The description and embodiments of the subject matter disclosed herein relate to systems and methods for calibrating a computing tomography (CT) imaging system, and in particular, a photon counting computed tomography (PCCT) system. In computed tomography (CT) imaging systems, an X-ray source or X-ray tube emits an X-ray beam towards an object, such as a patient, and X-rays attenuated by the subject are detected by one or more detectors (e.g., a detector array) to generate projection data that is used to reconstruct one or more images. The X-ray detector or detector array typically includes a collimator for collimating X-ray beams received at the detector, a scintillator disposed adjacent to the collimator for converting X-rays to light energy, and photodiodes for receiving the light energy from the adjacent scintillator and producing electrical signals therefrom. An intensity of the attenuated X-ray beam radiation received at the detector array is typically dependent upon the attenuation of the X-ray beam by the patient. Each detector element of a detector array produces a separate electrical signal indicative of the attenuated beam received by each detector element. The electrical signals are transmitted to a data processing system for analysis. The data processing system processes the electrical signals to facilitate generation of an image. Generally, in CT systems the X-ray source and the detector array are rotated about a gantry within an imaging plane and around the patient, and images are generated from projection data at a plurality of views at different view angles. For example, for one rotation of the X-ray source, 1000 views may be generated by the CT system.

Such conventional CT imaging systems utilize detectors that convert radiographic energy into current signals that are integrated over a time period, then measured and ultimately digitized. However, a drawback of such detectors is their inability to provide data or feedback as to the number and/or energy of photons detected. That is, the light emitted by the scintillator is a function of both a number of X-rays impinged and an energy level of the X-rays. The photodiodes may not be capable of discriminating between the energy level or the photon count from the scintillation. For example, two scintillators may illuminate with equivalent intensity and, as such, provide equivalent output to their respective photodiodes. Yet, despite yielding an equivalent light output, the number of X-rays received by each scintillator may be different, and an intensity of the X-rays may be different.

In contrast, PCCT detectors may provide photon counting and/or energy discriminating feedback with high spatial resolution. PCCT detectors can be caused to operate in an X-ray counting mode, and also in an energy measurement mode of each X-ray event. While a number of materials may be used in the construction of a direct conversion energy discriminating detector, semiconductors have been shown to be one preferred material. Typical materials for such use include Cadmium Zinc Telluride (CZT), Cadmium Telluride (CdTe) and Silicon (Si), which have a plurality of pixilated anodes attached thereto.

CT imaging systems, including PCCT systems, may demand relatively regular calibration scans, such as daily or weekly calibration scans. The calibration scans may include scanning a phantom or imaging phantom of a known composition, often a cylinder, plate, or slab with densities of already known values. The phantom is scanned and the PCCT system response is used to ensure that reconstructed images of the phantom have correct density values. Corrections may be made based on these values. Further, PCCT systems may obtain spectral information that allows generation of basis material decomposition (BMD) images. Calibrating PCCT systems may thereby demand that calibration projection data be obtained that mimics the materials and material thicknesses of the human body. Thus, phantoms for calibrating PCCT systems may include multiple different materials with nearly constant pathlengths across the spatial extent of the detector, such as polyvinyl chloride (PVC) and polyethylene (PE). In some examples, a phantom may include a series of uniform slabs of PVC or PE that may be scanned by the PCCT system to calibrate the PCCT system. However, a uniform phantom design may not provide an appropriately accurate calibration scan when the PCCT is being calibrated to detect small and/or low-contrast features in a clinical setting. Small and low-contrast features may be detected with high frequency x-rays and may be particularly impacted by non-ideal detector responses in a PCCT system. Non-ideal detector responses may include charge sharing and/or detector crosstalk. Charge sharing may occur when a single photon is converted to an electron-hole pair when the photon strikes the photon detector. The accumulation of electron-hole pairs may create a disperse charge cloud that may be detected by other pixels within the photon detector. Detector crosstalk refers to scattering of X-rays within the detector, which causes spatial and spectral blurring of measurements, for instance, of the X-ray photon counts in the PCCT detector. Both charge sharing and detector crosstalk can degrade the inherent resolution and contrast-to-noise ratio of a PCCT detector. Considering noteworthy or abnormal findings in medical images are often of relatively small geometric size and medium to low contrast with possible heterogenic surroundings, more accurate calibration of the response of the PCCT system may materially enhance the quality of images generated by the detector.

Thus, embodiments are disclosed herein for a non-uniform phantom and method for calibrating PCCT systems using the non-uniform phantom. In one embodiment, the non-uniform phantom may be a slab of material such as PVC or PE that includes a plurality of slots of various sizes, shapes and depths. The slots may be filled with a plurality of different materials. The size, shape, depth and material of the slots may simulate clinical features of interest to calibrate a PCCT scanner. In another embodiment, the non-uniform phantom may be cylindrical or elliptical in shape and include small beads of material (e.g., micro or sub-micro scale beads) of various materials and densities to simulate clinical features of interest. To calibrate a PCCT system, a uniform phantom may first be scanned by the PCCT system. Then, a non-uniform phantom may be placed inside the PCCT system and scanned from a plurality of angles and at a plurality of locations. The features of the non-uniform phantom may be well known, so an ideal response of the PCCT system may be predicted and compared to the actual response of the PCCT system, which may include blurring associated with detector crosstalk or charge sharing. A correction factor may then be calculated to adjust for the differences between the actual PCCT system response and the predicted PCCT system response, and a calibration algorithm may be adjusted based on the correction factor. The correction factor and corresponding calibration algorithm may be applied to future scans to increase the visibility of features similar to features included in the non-uniform phantom.

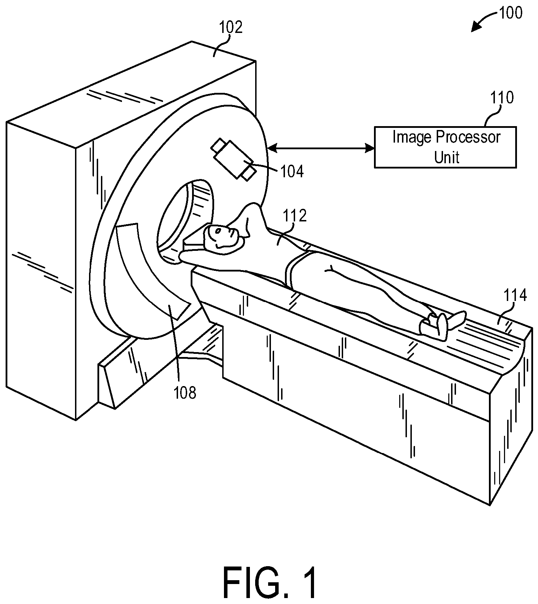

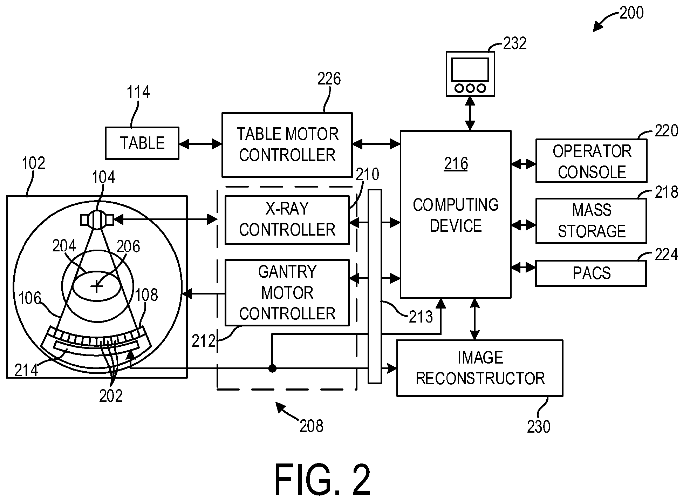

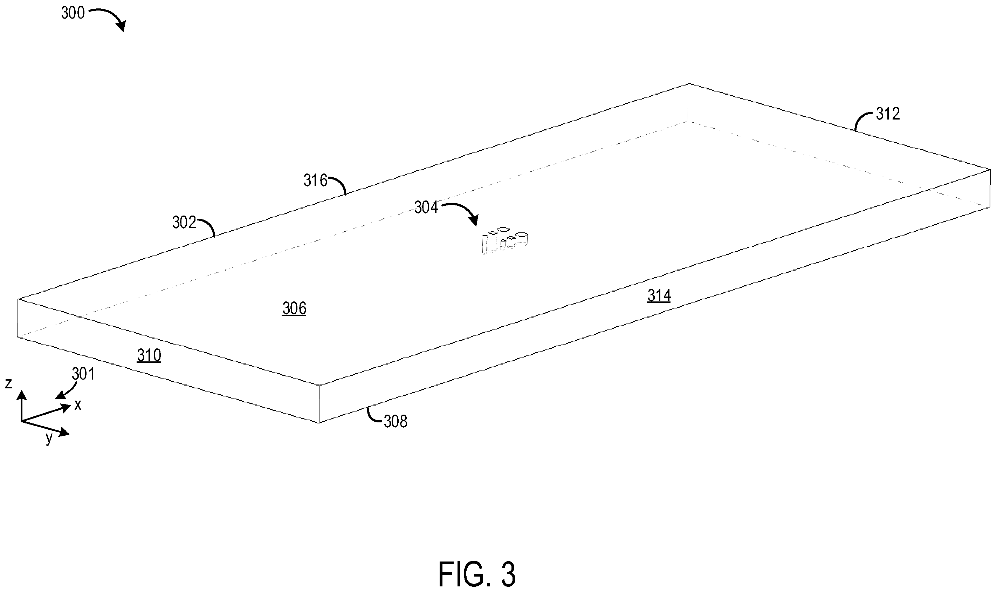

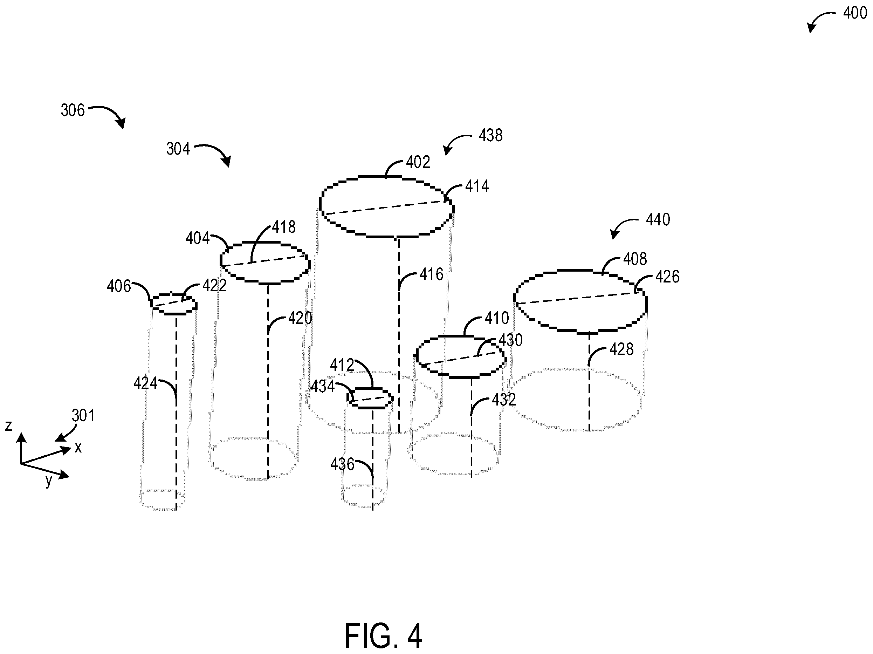

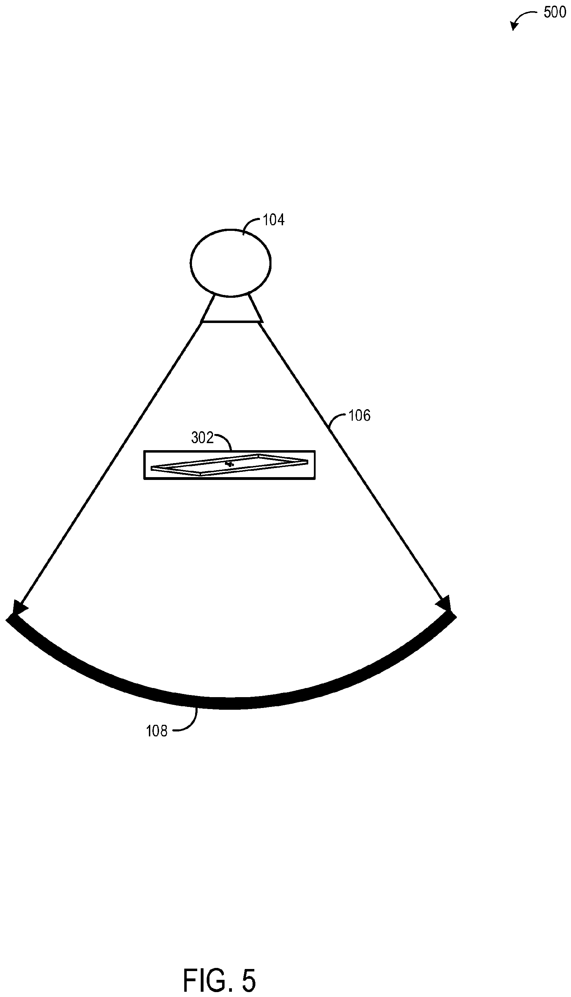

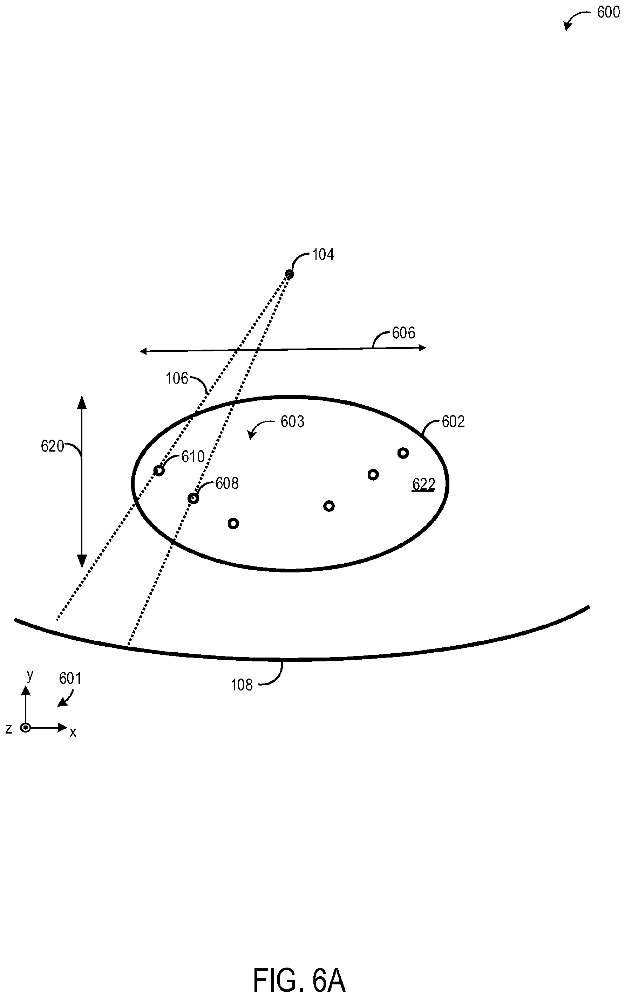

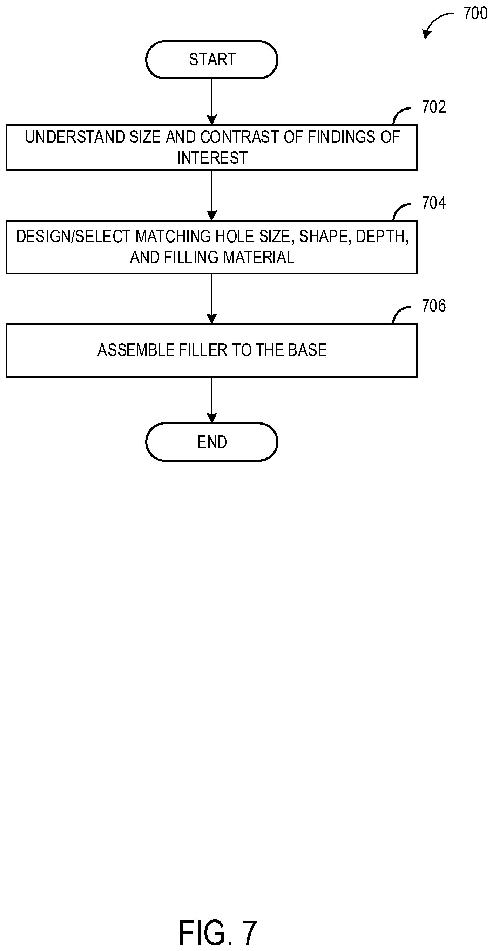

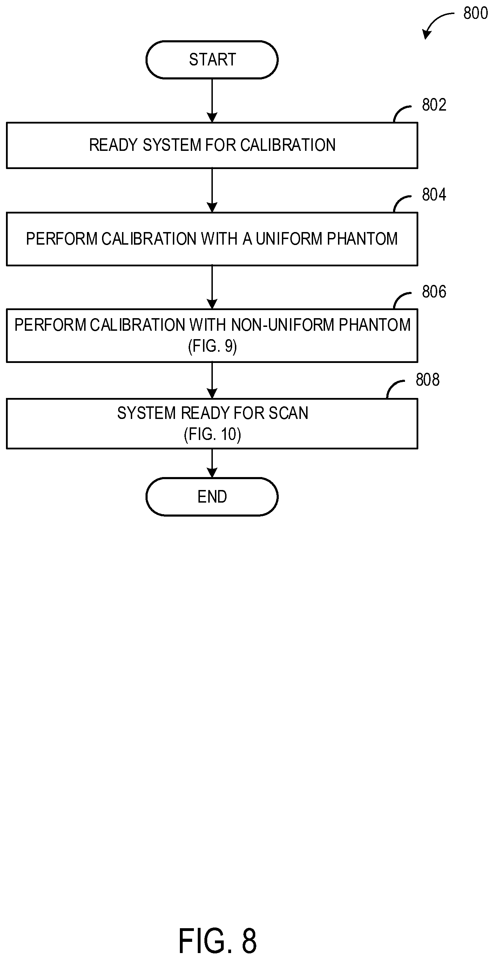

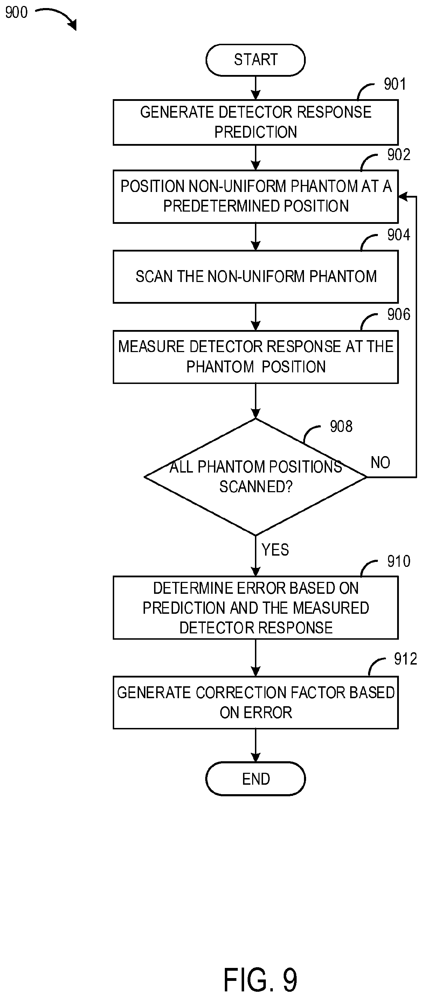

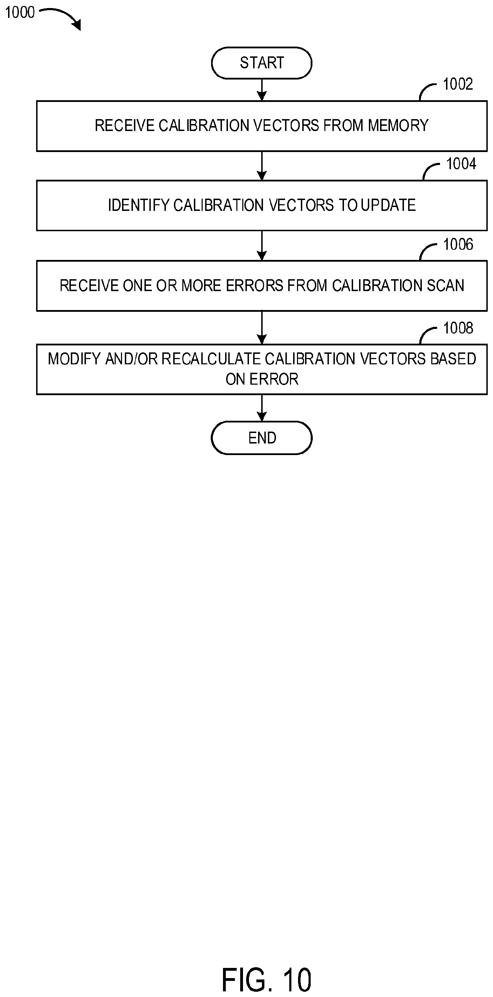

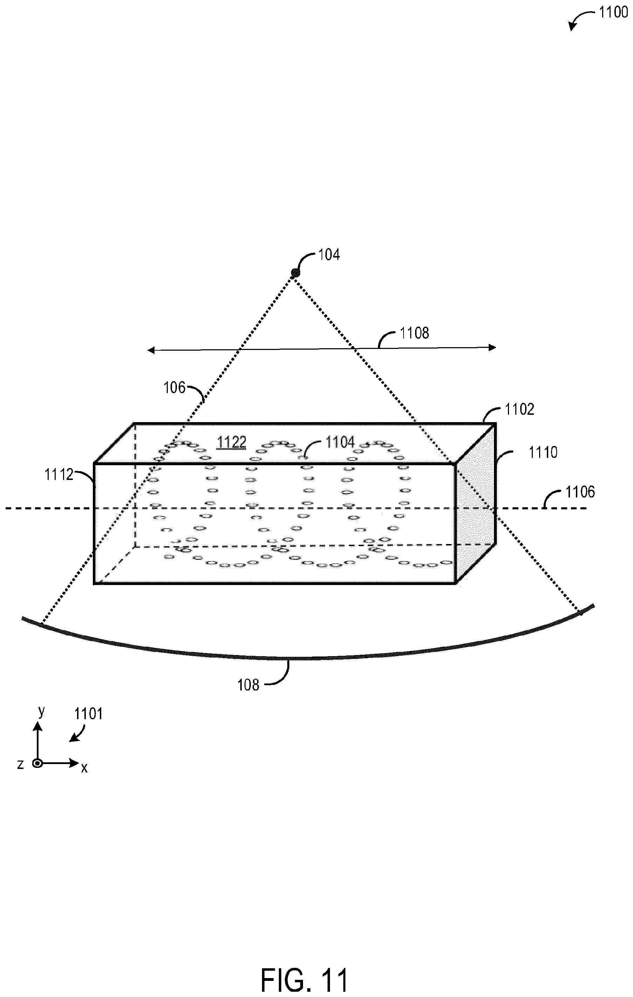

An example of a PCCT system including a PCCT scanner that may be used to perform imaging scans in accordance with the present techniques is provided in FIGS. 1 and 2. FIG. 3 shows an example detector array of the PCCT scanner, where photons of X-rays directed at a subject by an X-ray source are counted by detectors of the detector array. A first example of a non-uniform phantom that may be used in the disclosed calibration systems and methods is provided in FIG. 3. The non-uniform phantom may include one or more features of known composition embedded in a uniform base material. Examples of the features are shown in FIG. 4. FIG. 5 shows an example set up for performing the disclosed calibration methods. A second example of a non-uniform phantom, an elliptical base comprising micro or sub-micro beads, is provided in FIGS. 6A-C. Further examples of non-uniform phantoms are provided in FIGS. 11-14. Non-uniform phantoms may be customized to meet clinical requirements or research goals. An example workflow for designing a phantom is provided in FIG. 7. One example method for calibrating a PCCT system may include performing a calibration process with one or more uniform phantoms and one or more non-uniform phantoms, and adjusting the PCCT system based on the results of both calibrations, which is shown in FIG. 8. FIG. 9 shows an example method for performing a calibration using the disclosed non-uniform phantom. FIG. 10 shows an example method for applying calibration data to correct for detected error.

FIGS. 3-5 show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below/underneath one another, at opposite sides to one another, or to the left/right of one another may be referred to as such, relative to one another. Further, as shown in the figures, a topmost element or point of the element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example. FIGS. 6A-6B, and FIGS. 11-14 are schematic diagrams of example configurations with relative positioning of the various components.

FIG. 1 illustrates an exemplary PCCT system 100 (also referred to as a photon counting X-ray imaging system) configured for CT imaging with photon counting detectors. Particularly, the PCCT system 100 is configured to image a subject 112 such as a patient, an inanimate object, one or more manufactured parts, and/or foreign objects such as dental implants, stents, and/or contrast agents present within the body. The PCCT system 100 includes a gantry 102, which in turn, may further include at least one X-ray source 104 configured to project a beam of X-ray radiation 106 (see FIG. 2) for use in imaging the subject 112 laying on a table 114. Specifically, the X-ray source 104 is configured to project the beams of X-ray radiation 106 towards a detector array 108 positioned on the opposite side of the gantry 102. Although FIG. 1 depicts a single X-ray source 104, in certain embodiments, multiple X-ray sources and detectors may be employed to project a plurality of X-ray radiation beams for acquiring projection data at the same or different energy levels corresponding to the patient. In some embodiments, the X-ray source 104 may enable dual-energy gemstone spectral imaging (GSI) by rapid peak kilovoltage (kVp) switching. In the embodiments described herein, the X-ray detector employed is a photon counting detector which is capable of differentiating X-ray photons of different energies.

In certain embodiments, the PCCT system 100 further includes an image processor unit 110 configured to reconstruct images of a target volume of the subject 112 using an iterative or analytic image reconstruction method. For example, the image processor unit 110 may use an analytic image reconstruction approach such as filtered back projection (FBP) to reconstruct images of a target volume of the patient. As another example, the image processor unit 110 may use an iterative image reconstruction approach such as advanced statistical iterative reconstruction (ASIR), conjugate gradient (CG), maximum likelihood expectation maximization (MLEM), model-based iterative reconstruction (MBIR), and so on to reconstruct images of a target volume of the subject 112. In some examples the image processor unit 110 may use an analytic image reconstruction approach such as FBP in addition to an iterative image reconstruction approach.

In some CT imaging system configurations, an X-ray source projects a cone-shaped X-ray radiation beam which is defined with respect to an X-Y-Z Cartesian coordinate system and generally referred to as "imaging a volume." The X-ray radiation beam passes through an object being imaged, such as the patient or subject. The X-ray radiation beam, after being attenuated by the object, impinges upon an array of detector elements. The intensity of the attenuated X-ray radiation beam received at the detector array is dependent upon the attenuation of an X-ray radiation beam by the object. Each detector element of the array produces a separate electrical signal that is a measurement of the X-ray beam attenuation at the detector location. The attenuation measurements from all the detector elements are acquired separately to produce a transmission profile.

In some CT systems, the X-ray source and the detector array are rotated with a gantry within the imaging volume and around the object to be imaged such that an angle at which the X-ray beam intersects the object constantly changes. A group of X-ray radiation attenuation measurements, e.g., projection data, from the detector array at one gantry angle is referred to as a "view." A "scan" of the object includes a set of views made at different gantry angles, or view angles, during one or more revolutions of the X-ray source and detector.

FIG. 2 illustrates an exemplary imaging system 200 similar to the PCCT system 100 of FIG. 1. In accordance with aspects of the present disclosure, the imaging system 200 is configured for imaging a subject 204 (e.g., the subject 112 of FIG. 1). During certain scans, the subject may be a phantom. A phantom may be an object configured to be scanned by the PCCT system as part of a calibration process for the PCCT system. In one embodiment, the imaging system 200 includes the detector array 108 (see FIG. 1). The detector array 108 further includes a plurality of detector elements 202 that together sense the beam of X-ray radiation 106 (see FIG. 2) that passes through the subject 204 (such as a patient) to acquire corresponding projection data. In some embodiments, the detector array 108 may be fabricated in a multi-slice configuration including the plurality of rows of cells or detector elements 202, where one or more additional rows of the detector elements 202 are arranged in a parallel configuration for acquiring the projection data. The detector elements 202 may also be referred to as pixels or detector pixels.

In certain embodiments, the imaging system 200 is configured to traverse different angular positions around the subject 204 for acquiring desired projection data. Accordingly, the gantry 102 and the components mounted thereon may be configured to rotate about a center of rotation 206 for acquiring the projection data, for example, at different energy levels. Alternatively, in embodiments where the projection angle relative to the subject 204 varies as a function of time, the mounted components may be configured to move along a general curve rather than along a segment of a circle.

As the X-ray source 104 and the detector array 108 rotate, the detector array 108 collects data of the attenuated X-ray beams. The data collected by the detector array 108 undergoes pre-processing and calibration to condition the data to represent the line integrals of the attenuation coefficients of the subject 204. The processed data are commonly called projections. In some examples, the individual detectors or detector elements 202 of the detector array 108 may include photon counting detectors which register the interactions of individual photons into one or more energy bins.

The acquired sets of projection data may be used for basis material decomposition (BMD). During BMD, the measured projections are converted to a set of material-density projections. The material-density projections may be reconstructed to form a set of material-density maps or images of each respective basis material, such as bone, soft tissue, and/or contrast agent maps. The density maps or images may be, in turn, associated to form a 3D volumetric image of the basis material, for example, bone, soft tissue, and/or contrast agent, in the imaged volume.

Once reconstructed, the basis material image produced by the imaging system 200 reveals internal features of the subject 204, expressed in the densities of two basis materials. The density image may be displayed to show these features. In traditional approaches to diagnosis of medical conditions, such as disease states, and more generally of medical events, a radiologist or physician would consider a hard copy or display of the density image to discern characteristic features of interest. Such features might include lesions, sizes and shapes of particular anatomies or organs, and other features that would be discernable in the image based upon the skill and knowledge of the individual practitioner.

In one embodiment, the imaging system 200 includes a control mechanism 208 to control movement of the components such as rotation of the gantry 102 and the operation of the X-ray source 104. In certain embodiments, the control mechanism 208 further includes an X-ray controller 210 configured to provide power and timing signals to the X-ray source 104. Additionally, the control mechanism 208 includes a gantry motor controller 212 configured to control a rotational speed and/or position of the gantry 102 based on imaging requirements.

In certain embodiments, the control mechanism 208 further includes a data acquisition system (DAS) 214 configured to sample analog data received from the detector elements 202 and convert the analog data to digital signals for subsequent processing. The DAS 214 may be further configured to selectively aggregate data from a subset of the detector elements 202 into so-called macro-detectors. The data sampled and digitized by the DAS 214 is transmitted to a computer or computing device 216 via a slip ring 213. In one example, the computing device 216 stores the data in a storage device 218. The storage device 218, for example, may be any type of non-transitory memory and may include a hard disk drive, a floppy disk drive, a compact disk-read/write (CD-R/W) drive, a Digital Versatile Disc (DVD) drive, a flash drive, and/or a solid-state storage drive.

Additionally, the computing device 216 provides commands and parameters to one or more of the DAS 214, the X-ray controller 210, and the gantry motor controller 212 for controlling system operations such as data acquisition and/or processing. In certain embodiments, the computing device 216 controls system operations based on operator input. The computing device 216 receives the operator input, for example, including commands and/or scanning parameters via an operator console 220 operatively coupled to the computing device 216. The operator console 220 may include a keyboard (not shown) or a touchscreen to allow the operator to specify the commands and/or scanning parameters.

Although FIG. 2 illustrates one operator console 220, more than one operator console may be coupled to the imaging system 200, for example, for inputting or outputting system parameters, requesting examinations, plotting data, and/or viewing images. Further, in certain embodiments, the imaging system 200 may be coupled to multiple displays, printers, workstations, and/or similar devices located either locally or remotely, for example, within an institution or hospital, or in an entirely different location via one or more configurable wired and/or wireless networks such as the Internet and/or virtual private networks, wireless telephone networks, wireless local area networks, wired local area networks, wireless wide area networks, wired wide area networks, etc.

In one embodiment, for example, the imaging system 200 either includes, or is coupled to, a picture archiving and communications system (PACS) 224. In an exemplary implementation, the PACS 224 is further coupled to a remote system such as a radiology department information system, hospital information system, and/or to an internal or external network (not shown) to allow operators at different locations to supply commands and parameters and/or gain access to the image data.

The computing device 216 uses the operator-supplied and/or system-defined commands and parameters to operate a table motor controller 226, which in turn, may control a table 114 which may be a motorized table. Specifically, the table motor controller 226 may move the table 114 for appropriately positioning the subject 204 in the gantry 102 for acquiring projection data corresponding to the target volume of the subject 204.

As previously noted, the DAS 214 samples and digitizes the projection data acquired by the detector elements 202. Subsequently, an image reconstructor 230 uses the sampled and digitized X-ray data to perform high-speed reconstruction. Although FIG. 2 illustrates the image reconstructor 230 as a separate entity, in certain embodiments, the image reconstructor 230 may form part of the computing device 216. Alternatively, the image reconstructor 230 may be absent from the imaging system 200 and instead the computing device 216 may perform one or more functions of the image reconstructor 230. Moreover, the image reconstructor 230 may be located locally or remotely, and may be operatively connected to the imaging system 200 using a wired or wireless network. Particularly, one exemplary embodiment may use computing resources in a "cloud" network cluster for the image reconstructor 230.

In one embodiment, the image reconstructor 230 stores the images reconstructed in the storage device 218. Alternatively, the image reconstructor 230 may transmit the reconstructed images to the computing device 216 to generate useful patient information for diagnosis and evaluation. In certain embodiments, the computing device 216 may transmit the reconstructed images and/or the patient information to a display or display device 232 communicatively coupled to the computing device 216 and/or the image reconstructor 230. In some embodiments, the reconstructed images may be transmitted from the computing device 216 or the image reconstructor 230 to the storage device 218 for short-term or long-term storage.

Information may be transmitted between the components residing in the gantry 102 and external devices (such as the computing device 216 and/or image reconstructor 230) via the slip ring 213, which facilitates electronic communication across the rotating gantry. In some examples, the gantry and internal components (e.g., the control mechanism 208, X-ray source 104, the detector array 108) may be collectively defined as a PCCT scanner, and as such the computing device 216 and image reconstructor 230 may reside off the scanner.

FIG. 3 is a schematic diagram 300 of an example phantom 302. The example phantom 302 is one example of a non-uniform phantom of the present disclosure. FIG. 3 includes a Cartesian coordinate system 301. The z-axis of coordinate system 301 may be a vertical axis (e.g., parallel to a gravitational axis), the y-axis of coordinate system 301 may be a longitudinal axis (e.g., horizontal axis), and/or the x-axis of coordinate system 301 may be a lateral axis, in one example. However, the axes may have other orientations, in other examples. When referencing direction, positive may refer to in the direction of the arrow of the x-axis, y-axis, and z-axis and negative may refer to in the opposite direction of the arrow of the x-axis, y-axis, and z-axis. A filled circle may represent an arrow and axis facing toward, or positive to, a view. An unfilled circle may represent an arrow and an axis facing away, or negative to, a view. Further, FIG. 3 is drawn to scale, though other relative dimensions could be used if desired.

The example phantom 302 may be a solid rectangular prism made out of a base material, and one or more known composition features embedded in the base material, the one or more known composition features comprising a different material than the base material. For example, the base material may include a plastic such as polyvinyl chloride (PVC) or polyethylene (PE). However other materials are possible, such as a metal. The phantom 302 may include a top 306, a bottom 308, a front 310, a back 312, a first side 314 and a second side 316. The top 306 and the bottom 308 may be rectangular faces in the x-y plane with a smaller width than length. The front 310 and the back 312 may be rectangular faces in the x-z plane with a length that matches the length of the top 306 and the bottom 308. The first side 314 and the second side 316 may be rectangular faces in the y-z plane with a length that matches the width of the length of the top 306 and the bottom 308. In one example, the phantom 302 may have a depth of between 2.5 mm and 5 mm.

The one or more known composition features include clinically relevant object sizes and contrasts. In some examples, the one or more known composition features may include one or more of a hole, slot, recess, or bore in the base material. As shown in the example, the top 306 may include a set of slots 304. In the example shown in FIG. 3, the set of slots 304 is located in the center of the top 306 and makes up a small portion of the area of the top 306. However, in other examples the set of slots 304 may be located at any position on the top 306 and may take up more of the surface of the top 306. The set of slots 304 is described in more detail with respect to FIG. 4.

Turning now to FIG. 4, a close up view 400 of the set of slots 304 included in the top 306 is shown. The example set of slots 304 shown in FIG. 4 may include a first slot 402, a second slot 404, a third slot 406, a fourth slot 408, a fifth slot 410, and a sixth slot 412. However, in other examples there may be more or fewer slots included in the set of slots 304. Each slot may be a cylindrical void in the top 306 of the phantom 302 and the slot size may be defined by a height and a diameter.

The slots 304 may be filled with a filler material. In some examples, the slot may be filled with a cylinder of equivalent size and shape to the slot made of a material used to simulate a feature of clinical relevance. For example, a slot may be filled with a material of a known contrast, a plastic such as PVC or PVE, or a metal such as aluminum, cadmium or tungsten. The material used to fill a slot imaged against the material of the phantom 302 may produce different levels of contrast in the resulting image. The material used to fill a slot may be selected in order to produce different levels of contrast, which may be used to calibrate the PCCT system.

The slots 304 may be of a known size, a known height, a known diameter, and may be arranged at a known depth within a base material. Additionally, the size of each slot and the material used to fill the slot may be varied to mimic the size of clinical features. The diameter and height of each slot are represented by dashed lines in FIG. 4. The diameter may define the size of a circular opening in the top 306 that defines the top of each slot. The height may define the depth that the slot extends into the phantom 302 from the top 306. The slots heights may be anywhere from 25%-100% of the height of the phantom 302.

The first slot 402 may have a first diameter 414 and a first height 416; the second slot 404 may have a second diameter 418 and a second height 420; the third slot 406 may have a third diameter 422 and a third height 424; the fourth slot 408 has a fourth diameter 426 and a fourth height 428; the fifth slot 410 has a fifth diameter 430 and a fifth height 432; and the sixth slot 412 has a sixth diameter 434 and a sixth height 436. In the example set of slots 304, the slots may be arranged in two rows along the x-axis and separated by a distance in the y-direction. A first row 438 may contain the first slot 402, the second slot 404, and the third slot 406. A second row 440 may contain the fourth slot 408, the fifth slot 410, and the sixth slot 412. In the example set of slots 304 shown in FIG. 4, the slots within the first row 438 may share a height and the slots within the second row 440 may share a different height. In other words, the first height 416, the second height 420, and the third height 424 may all be equal. The fourth height 428, the fifth height 432 and the sixth height 436 may all be equal and may be shorter than the height of slots within the first row 438. However, the heights of the slots may be distributed differently in other example sets of slots. The first row 438 and the second row 440 may contain slots that have the same diameter. The first diameter 414 may be equivalent to the fourth diameter 426, the second diameter 418 may be equivalent to the fifth diameter 430, and the third diameter 422 may be equivalent to the sixth diameter 434. Varying slot sizes may allow the PCCT system to be calibrated to image features that may be similar in diameter to one or more of the slots and may have a depth similar to one or more slots. In the example described above, the set of slots 304 are described as slots, however in other examples they may be holes, recesses, or bores.

The example phantom 302 is one example of a non-uniform phantom. Numerous configurations are possible, and the design of the non-uniform phantom, including the base material and the one or more known composition features, may be selected based on one or more of a calibration method, a clinical purpose, and a research purpose. One example of a workflow for designing a non-uniform phantom is described below with reference to FIG. 7.

FIG. 5 depicts a use example 500 of a non-uniform phantom for calibrating a PCCT system, such as the example phantom 302 in FIGS. 1 and 3 and the PCCT system 100, respectively. The phantom 302 is placed within the gantry 102 of a PCCT system. The phantom 302 may be positioned underneath the X-ray source 104. As described in more detail with respect to FIG. 2, the X-ray source 104 may emit the beam of X-ray radiation 106. In some examples, the beam of x-ray radiation 106 may be conical in shape. The phantom 302 may be positioned to intercept the beam of x-ray radiation 106. In one example, the phantom 302 may be placed atop the table 114 and the patient table may be moved into the path of the beam of X-ray radiation 106. In other examples, the phantom 302 may be coupled to a holder that projects outward from the patient table. This may allow the phantom 302 to be placed in the path of the beam of X-ray radiation 106 without the patient table interrupting the beam of X-ray radiation 106. Removing the influence of the patient table may remove the influence of the table on the detector response, error calculations and calibration algorithms. X-rays that impinge upon the phantom 302 may be attenuated by the material within the phantom 302 and strike the detector array 108. The detector array 108 may respond to being struck by attenuated X-rays and the response may be measured according to the method described with respect to FIG. 2. During a calibration process, the phantom 302 may be placed in different positions and scanned repeatedly. In some examples, the phantom 302 may be placed in positions that cover the entirety of the detector array 108 and scanned. Scanning the phantom 302 in positions across the entire detector array 108 may allow the PCCT system 100 to be calibrated to detect clinical features of interest at any position relative to the detector array 108. In addition, the phantom 302 may be placed at different distances from the X-ray source 104 and the detector array 108. Placing the phantom 302 at different distances from the detector array 108 may affect the focal spot-induced blurring in the images produced by the PCCT 100. The closer the phantom 302 is to the detector array 108, the less focal spot blurring may be present in the final images. The phantom 302 may be placed at different distances from the detector array 108 to cover the whole detector; and at different distances to measure different levels of focal spot blurring.

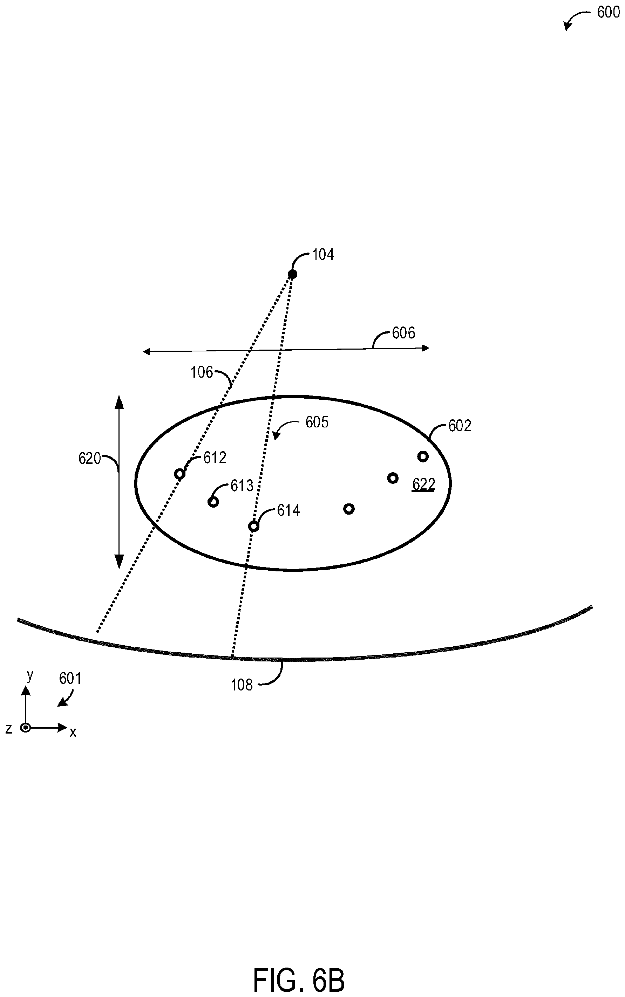

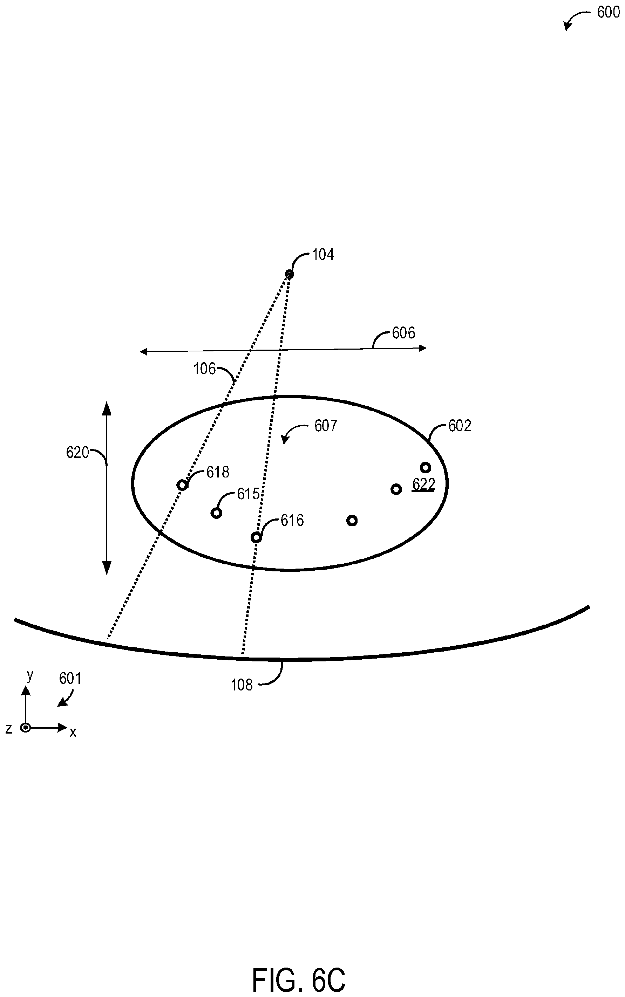

FIG. 6A, 6B, and 6C depict a schematic diagram 600 of a second example phantom 602. The FIGS. 6A, 6B, and 6C include a Cartesian coordinate system 601. The y-axis of coordinate system 601 may be a vertical axis (e.g., parallel to a gravitational axis), the x-axis of coordinate system 601 may be a longitudinal axis (e.g., horizontal axis), and/or the z-axis of coordinate system 601 may be a lateral axis, in one example. However, the axes may have other orientations, in other examples. When referencing direction, positive may refer to in the direction of the arrow of the x-axis, y-axis, and z-axis and negative may refer to in the opposite direction of the arrow of the x-axis, y-axis, and z-axis. A filled circle may represent an arrow and axis facing toward, or positive to, a view. An unfilled circle may represent an arrow and an axis facing away, or negative to, a view.

FIGS. 6A, 6B, and 6C represent cross-sectional views at different z-positions of a second phantom 602 with an elliptical shape. The second phantom 602 may be elliptical in shape with a length 606 along the x-axis that in one example may be 40 cm. The second phantom 602 may have a width 620 along the y-axis that is less than the length 606. The second phantom 602 may be positioned between the X-ray source 104 and the detector array 108 such that the X-ray beam 106 from the X-ray source 104 may be attenuated by the second phantom 602 and strike the detector array 108. The second phantom 602 may comprise a base material 622 and one or more known composition features arranged in the base material, the one or more known composition features comprising a different material than the base material 622. For example, the base material 622 may include any of the aforementioned base materials or other clinically relevant materials, e.g., PVC, PE, metal, air, and so on. The one or more known composition features may include a plurality of beads of material that may mimic clinical findings. For example, the beads may be of a plurality of sizes, and may include micro- or nano-beads. The beads may be made of a various materials and densities. For example, the beads may be made of hard metals to represent medical implants or the beads may be made of calcium to mimic plaque within a body. In other examples the beads may be made of iodine, solid water at different densities, PVC or PE. A phantom such as the second phantom 602 may include one or more size and material of bead. In some examples, the beads may be uniformly spaced and/or spaced in a pattern throughout the volume of a phantom. In other examples, the beads may be distributed randomly throughout a phantom. In example phantoms including one or more sized and/or material of bead, the different sizes and/or materials of bead may also be distributed in a pattern or randomly.

FIG. 6A depicts a cross sectional view of an example phantom in an x-y plane at a first position along the z-axis. At the first z-position, the second phantom 602 includes a first set of beads 603. The first set of beads 603 may be arranged in an arbitrary pattern in the x-y plane, or they may be arranged in two diagonal lines that each include three beads. The beads included in each diagonal line may be evenly spaced or unevenly spaced. In the view of the second phantom 602 provided by FIG. 6A the X-ray beam 106 may include a first bead 610 and a second bead 608, and may be imaged by the detector array 108. However, in other examples, the X-ray beam 106 may be adjusted to include one or more adjacent beads of the first set of beads 603.

FIG. 6B depicts a cross sectional view of an example phantom in an x-y plane at a second position along the z-axis. The second position may be in a more negative position along the z-axis than the first position. At the second z-position, the second phantom 602 includes a second set of beads 605. The second set of beads 605 may be arranged in an arbitrary pattern in the x-y plane, or they may be arranged in two diagonal lines that each include three beads. The second set of beads 605 may not be arranged in the same x-y positions as the first set of beads 603. The beads included in each diagonal line may be evenly spaced or unevenly spaced. In the view of the second phantom 602 provided by FIG. 6B the X-ray beam 106 may span from a third bead 612 to a fourth bead 614 and include a fifth bead 613 within the X-ray beam. The third bead 612, the fourth bead 614 and the fifth bead 613 may be imaged by the detector array 108. However, in other examples, the X-ray beam 106 may be adjusted to include one or more adjacent beads of the second set of beads 605.

FIG. 6C depicts a cross sectional view of a phantom in an x-y plane at a third position along the z-axis. The third position may be in a more negative position along the z-axis than the second position. At the third z-position, the second phantom 602 includes a third set of beads 605. The second set of beads 607 may be arranged in an arbitrary pattern in the x-y plane, or they may be arranged in two diagonal lines that each include three beads. The third set of beads 607 may not be arranged in the same x-y positions as the first set of beads 603 or the second set of beads 605. The beads included in each diagonal line may be evenly spaced or unevenly spaced. In the view of the second phantom 602 provided by FIG. 6C the X-ray beam 106 may span from a sixth bead 618 to a seventh bead 616 and include an eighth bead 615 within the X-ray beam. The sixth bead 618, the seventh bead 616 and the eighth bead 615 may be imaged by the detector array 108. However, in other examples, the X-ray beam 106 may be adjusted to include one or more adjacent beads of the third set of beads 607. There may be further possible embodiments of a phantom that includes beads of material, which are described in more detail with respect to FIGS. 11-14.

FIG. 7 depicts a flowchart that describes a workflow 700 for designing a non-uniform phantom such as phantom 302 that includes a plurality of slots such as the set of slots 304 or features. The workflow 700 may include selecting a base material, and one or more features of known composition that may be embedded in the base material, including, but not limited to a filling material, a feature shape, size and depth. The workflow 700 may be tailored to the goal of the non-uniform phantom, including but not limited to, calibration, correction, or evaluation of the detector, clinical purpose, and research purpose.

At 702, the workflow 700 may include understanding the clinical features of interest the PCCT is being calibrated to identify. The size, material, and contrast of the features may be understood through clinical study.

At 704, the workflow 700 may include designing or selecting a hole size, shape, depth and filling material for the phantom that matches the clinical features of interest. For example, based on the clinical features of interest, one or more objects of a known height, a known diameter, a known contrast, or other relevant parameters may be incorporated into the design. The phantom may be made of a first material, e.g., the base material, and include a plurality of slots that a second material may be inserted into. The contrast between the first material and the second material may vary based on the composition of the second material. In one example, the slots may be filled with air to provide low contrast, and in another example they may be filled with PVC to provide high contrast. In other examples, the slots may be filled with of any one or more of a pure, composite, doped, coated, organic, inorganic, solid, or fluid material. For example, the non-uniform phantom may comprise PE as the base material and iodine as the filling material. In some examples, the base material may be configured as a slab, such as the example given in FIGS. 3-4, however other examples may include a cylindrical or elliptical base material. In yet another example, the base material may be air, and the filler can be some more attenuating material. Such an example may include a standalone small object as the phantom without the slab base material.

Furthermore, the depth of the slot may simulate different levels of contrast. The deeper the slot, the higher the contrast level. The slots may be of a plurality of sizes and may range in size between 0.1 mm and 100 mm to match a range of clinical features of interest. The slots may be cylindrical in one example, but could be rectangular, square or irregular in other examples. In some examples, the phantom may contain one or more patterns of slots. The feature can also include patterns of holes or slots. A pattern may include one or more slots arranged in a particular configuration, arrangement, or shape. The slots may be of any size or shape, and a single pattern may contain one or more distinct size and shape of slot. Patterns may be located in different regions of the phantom (e.g., center, edges, corners), uniformly distributed across the phantom, or any other distribution that matches the clinical goals of the calibration. For example, the non-uniform phantom may include one pattern or multiple patterns. If multiple patterns are included, the patterns may be the same pattern repeating or different patterns. Further, a number of slots or other features of known composition may vary, e.g., 1 slot, 10 slots, 100 slots, etc. Each pattern can be an arrangement of 1 or multiple holes or slots, and the holes or slots can be of different shapes and depths.

At 706, the workflow 700 may include assembling the filler to the base. The filler may be the second material that is inserted into slots, while the base may be the body of the phantom made of the first material that includes slots formed into the body. The filler may be assembled into the base by a variety of methods. In one example, one or more solid plugs of material that match the dimensions of the slots may be inserted into one or more slots. In another example, a liquid material may be poured into one or more slots. In some examples, the slots may be of a standardized size or shape so that plugs of material, also of a standardized size, may be interchangeable to assemble different material combinations.

FIG. 8 depicts a flowchart that describes a method 800 for performing a calibration using a non-uniform phantom such as phantom 302 and a uniform phantom. Method 800 may be carried out according to instructions stored in memory of one or more controllers or computing devices included as part of and/or operatively coupled to a CT imaging system, such as DAS 214, X-ray controller 210, image reconstructor 230, and/or computing device 216.

At 802, the method 800 may include readying the system for calibration. Readying the system for calibration may include powering on the PCCT system and adjusting the settings of the PCCT system to produce an x-ray beam that is suitable for calibration.

At 804, the method 800 may include performing a calibration on a uniform phantom. The uniform phantom may be comprised of a combination of materials with a uniform path length across the spatial extent of the detector. For example, a uniform phantom may be a slab of PVC that covers the spatial extent of the detector. In another example, the uniform phantom may include one or more uniform slabs of PE stacked on top of a slab of PVC. The uniform slabs may effectively calibrate the PCCT system to create a basic model based correction factor. The model based correction factor may be improved by following the uniform calibration with a calibration using a non-uniform phantom. For example, following a calibration using a uniform phantom with a non-uniform phantom calibration may enable computing a contrast of the inserts or plugs arranged in the slots of the non-uniform phantom. For example, to compute contrast, the process may include comparing the measured intensity of a region of interest (e.g., the inserts or plugs) against a region in the background. The background may be obtained from the uniform slab. Such approaches to calibration enable fine tuning of correction models and increase performance of scanning non-uniform objects in practice. One example of performing a calibration with a uniform phantom may include placing one or more uniform phantoms within the gantry of a PCCT system and in the path of an X-ray beam and scanning the uniform phantom. The phantom may be scanned at one or more distances from the detector array during a calibration process. One or more uniform phantoms may be scanned during a calibration process. The calibration process may include applying one or more protocols such as different combinations of voltage, current, and slice thickness, and acquiring images. The images may be analyzed according to measurements of image quality metrics, such as focal spot-induced blurring, noise level and contrast-to-noise ratio. There may be a ground truth image comprised of the expected scan results based on the specifications of the PCCT system and the qualities of the uniform phantom. One or more correction factors may be calculated based on the data obtained and the expected results. In some examples, a model based algorithm may be used to generate a correction factor based on the ground truth definition of the phantoms and all blurring phenomena for each scan performed during the calibration process. Blurring phenomena may include focal spot blurring. The uniform phantom calibration data may be used to validate models of the spectral response of the detector and to compute contrast and contrast-to-noise ratio values. The contrast and contrast to noise ratio values can be used to evaluate a detector scatter correction algorithm after calibrating said algorithm to reduce errors based on known ground-truth behavior of detector scatter for the non-uniform phantoms.

At 806 the method 800 may include performing a calibration process on a non-uniform phantom such as phantom 302. The calibration process for a non-uniform phantom is described in more detail with respect to FIG. 9. Briefly, the calibration process may include placing the non-uniform phantom at a position within the PCCT scanner, scanning the non-uniform phantom, and determining the focal spot blurring in the image resulting from the scan for one or more non-uniform phantom positions. Once the non-uniform phantom has been scanned at all of the required positions, error terms may be determined for each scan result and used to generate one or more correction factors. The correction factors may be applied directly to acquired image data during image reconstruction of subject scans. Additionally, or alternatively, the correction factors may be used to adjust one or more calibration algorithms, such as a crosstalk correction algorithm or a charge sharing correction algorithm.

At 808, the method 800 may determine the system is ready for scanning a subject. For example, with the system ready, the method 800 may include applying the calibration data to perform a scan of a subject. For example, one or more adjusted calibration algorithms may be implemented in image reconstruction of the scanned subject. Similarly, the method 800 may include applying one or more adjusted correction factors to perform a scan of a subject. An example method for applying calibration data to correct for detected error is described in more detail with respect to FIG. 10. The correction factors determined during the calibration performed on a uniform phantom and the calibration performed on a non-uniform phantom may be applied to all targets that are scanned with the PCCT system. The calibration performed on the non-uniform target may increase the quality of images produced by the PCCT system and may particularly increase the quality of images of clinical targets that may be small in size, have low contrast against surrounding material, and clinical targets that may be located against a heterogeneous background.

FIG. 9 is a flowchart describing a method 900 for obtaining correction factors by performing a calibration based on a non-uniform phantom such as phantom 302. Method 900 may be carried out according to instructions stored in memory of one or more controllers or computing devices included as part of and/or operably coupled to a CT imaging system, such as DAS 214, X-ray controller 210, image reconstructor 230, and/or computing device 216. Method 900 may be carried out as part of method 800. For example, method 900 may be carried out at 806 of method 800. In one example, the method 900 may be carried out for correcting one or more of charge sharing, detector crosstalk, bowtie scatter, object scatter, focal spot movement, and similar phenomena. Additionally, the method 900 may be used to validate models for spectral response of the PCCT detector.

At 901, the method 900 may include generating a detector response prediction for one or more known composition features of the non-uniform phantom. In one example, generating the detector response prediction may include calculating a contrast prediction based on a material composition of the one or more known composition features, relative to the material composition of the base material (e.g., from the uniform phantom). The detector response prediction may be related to a ground truth of the known composition features and the base material comprising the non-uniform phantom or the base material comprising the uniform phantom. For example, the detector response prediction may be derived from the dimensions, density, and position of the features selected to simulate a contrast level whose ground-truth value is known by design. For example, the phantom may be made of one or more materials of a predictable attenuation contrast, which may be used to predict an expected contrast in images resulting from a scan.

At 902, the method 900 may include positioning the non-uniform phantom at a predetermined position. The phantom may be placed on or attached to a patient table such as table 114. The table may be motorized, which may allow the position of the table, and by extension the non-uniform phantom, to be adjusted remotely. Remote adjustment of the non-uniform phantom may allow the non-uniform phantom to be moved into one or more preset positions, which may allow the position of the non-uniform phantom to be replicated between calibrations.

At 904, the method 900 may include scanning the non-uniform phantom. Scanning the non-uniform phantom may include directing a beam of X-rays at the non-uniform phantom. The X-rays may be attenuated by the non-uniform phantom and detected by a photon counting detector array. The X-ray source and detector array may be rotated in order to image the non-uniform phantom from a plurality of angles. At 906, the method may include measuring the detector response at the phantom position. In one example, measuring the detector response may include measuring focal spot blurring at the phantom position. Focal spot blurring may be the degree to which the image is blurred due to x-rays being produced at slightly different positions within a finite focal spot. Additionally, blurring due to charge sharing and detector crosstalk may also be measured at 906.

At 908, the method may include determining if all non-uniform phantom positions have been scanned. The calibration process may include positioning the non-uniform phantom at a plurality of preset distances from the detector array and performing scans at each distance. If all non-uniform phantom positions have not been scanned at 908, the non-uniform phantom may be moved to a new position at 902. If all of the phantom positions have been scanned at 908, at 910 the method may include determining an error measurement based on the prediction and the actual system response to a scan. Otherwise, the method 900 continues to scan at 902 the non-uniform phantom. One example of the error measurement may include a measured amount of focal spot blurring. Another example of the error measurement may include measuring the blur induced by detector crosstalk, charge sharing and other phenomena that contribute to blur by comparing ground-truth projections computed using physics-based models for the known features of the non-uniform phantom to measurements obtained from the non-uniform phantom. The error measurement may be determined based on differences between the ground truth of the known composition features and the images that result from the scans, including, but not limited to, the ground truth of the size, material density, expected contrast, and so on, compared to the measured size, measured density, contrast, and so on.

At 912, the method 912 may include generating a correction factor based on the error measurement. The correction factor may be generated by one or more algorithms and may be calculated by one or more computers or processors. Further, the correction factor may be used to adjust one or more parameters of the calibration algorithms to reduce error. In an example where error is due to detector crosstalk and charge sharing, a correction factor may be calculated based on the error that proposes adjustments to the parameters of the theoretical physics-based model parameters such as the strength of the estimated crosstalk and charge sharing. The adjusted physics-based model is thereby tuned to minimize error when reconstructing images from spectral data obtained from scanning a target. In one example, the algorithm may be a model-based detector crosstalk and a model-based detector crosstalk and charge sharing correction algorithm. In one example, the algorithm may be patient/scanner scatter correction algorithm. In another example, the correction algorithm may be a spectral correction algorithm that produces monochromatic images or material images. In some examples, the correction factor may vary depending on the distance of a subject from the detector array. When the correction factor is applied to a scan, the resulting image may have clearer contrast between low-contrast features and the background, increased resolution to see smaller features, and increased visibility of features against a heterogeneous background. Another application of the non-uniform phantom may include directly measuring the phantoms for image quality assessment.

FIG. 10 shows a flowchart describing a method 1000 for applying calibration data generated by the calibration methods of FIG. 8 and FIG. 9 to correct for the detected error. Method 1000 may be carried out according to instructions stored in memory of one or more controllers or computing devices included as part of and/or operably coupled to a CT imaging system, such as DAS 214, X-ray controller 210, image reconstructor 230, and/or computing device 216. In one example, method 1000 may be carried out as part of method 800. For example, method 1000 may be carried out at 808 of method 800. In one example, the method 1000 may be carried out for adjusting one or more algorithms. Such algorithms may include physics-based models that model the influence of phenomena such as detector crosstalk or charge sharing, spectral detector efficiency modelling, spectral correction algorithms and patient/scanner correction algorithms. Physics-based models may be able to reconstruct images from spectral data based on the physical properties of the phantom and modeling of physical phenomena such as detector crosstalk or charge sharing. Spectral detector efficiency models may be a physics-based model of the efficiency of the spectral detector, which may influence the detector response to the calibration phantom. Spectral correction algorithms may use models or calibration vectors to predict expected detector response from known material path lengths. Patient/scanner scatter correction algorithms may reduce material-dependent shading artifacts. Tuning the patient/scanner scatter correction algorithms using a non-uniform phantom may include determining the amount of materially-dependent error in scatter estimates and using the amount of error to tune the patient/scanner scatter correction algorithms.

At 1002, the method 1000 may include receiving calibration vectors representing detector elements of the PCCT system of from memory.

At 1004, the method 1000 may include identifying one or more calibration vectors to update. As a few non-limiting examples, one or more calibration vectors to update may be identified based on a calibration schedule, an error detection resulting from an imaging operation of the PCCT system, or an error detected during a calibration scan.

At 1006, the method 1000 may include receiving one or more errors from a calibration scan. An error may represent a discrepancy between a predicted system response and an actual system response. Further, the error may include a correction factor derived from the error based on a model of the PCCT system response. The error may be determined by first obtaining data from a scan of the non-uniform phantom. The data may then be processed by one or more physics-based models to reconstruct images of the non-uniform phantom. The error may be calculated based on a comparison between the reconstructed images if the non-uniform phantom and the ground truth features of the phantom.

At 1008, the method 1000 may include modifying and/or recalculating one or more calibration vectors based on the error. For example, if there is error between the reconstructed image of the non-uniform phantom and the ground truth properties of the non-uniform phantom due to underestimated detector crosstalk, one or more calibration vectors associated with correcting detector crosstalk may be adjusted to correct the error.

As one example, the data from the non-uniform phantom can be taken through a physics-model-based detector crosstalk correction and images reconstructed. If in the reconstructed images, the inserted structures do not exhibit the known ground-truth contrast, the physics model (constituting the crosstalk calibration vectors) used in detector crosstalk correction can be adjusted/tuned. For example, the library of materials for which the correction is designed to work can be expanded to include more materials or material combinations to ensure that the reconstructed images exhibit material properties close to the expected known ground-truth for the non-uniform phantom.

FIGS. 11-14 depict a variety of phantom designs that include beads of material arranged in an example spiral pattern within the phantom. FIGS. 11-14 include a Cartesian coordinate system 1101. The y-axis of coordinate system 1101 may be a vertical axis (e.g., parallel to a gravitational axis), the x-axis of coordinate system 1101 may be a longitudinal axis (e.g., horizontal axis), and/or the z-axis of coordinate system 1101 may be a lateral axis, in one example. However, the axes may have other orientations, in other examples. When referencing direction, positive may refer to in the direction of the arrow of the x-axis, y-axis, and z-axis and negative may refer to in the opposite direction of the arrow of the x-axis, y-axis, and z-axis. A filled circle may represent an arrow and axis facing toward, or positive to, a view. An unfilled circle may represent an arrow and an axis facing away, or negative to, a view.

FIG. 11 depicts a schematic diagram 1100 of first embodiment of a rectangular prism phantom 1102 that includes beads. The first embodiment of the rectangular prism phantom 1102 may be a rectangular prism with a length 1108 along the x-axis. In one example the length 1108 may be 40 cm. The first embodiment of a rectangular prism phantom 1102 may be positioned between the X-ray source 104 and the detector array 108 such that the X-ray beam 106 from the X-ray source 104 may be attenuated by the first embodiment of a rectangular prism phantom 1102 and strike the detector array 108.

The first embodiment of the rectangular prism phantom 1102 may have a rectangular front face 1112 and a rectangular back face 1110 that may lie in parallel y-z planes, separated by the length 1108. Rectangular faces may couple the front face 1112 and the back face 1110 along each of their respective sides to form a rectangular prism. In one example, the front face 1112 and the back face 1110 may be squares. A midline 1106 may run through the center of the front face 1112 and the back face 1110 along the x-axis. The rectangular prism phantom 1102 may comprise a base material 1122 and one or more known composition features arranged in the base material, the one or more known composition features comprising a different material than the base material 1122. For example, the base material 1122 may include any of the aforementioned base materials or other clinically relevant materials, e.g., PVC, PE, metal, air, and so on. The one or more known composition features may include a spiral of beads 1104 that may mimic clinical findings, such as the beads described in more detail with respect to FIGS. 6A-6C. The spiral of beads 1104 may form a circular spiral centered around the midline 1106. The radius of the spiral, number of beads within the spiral, size of bead and material of bead may or may not be held constant within the first embodiment of a rectangular prism phantom 1102. The length 1108 of the first embodiment of a rectangular prism phantom 1102 and the position of the first embodiment of a rectangular prism phantom 1102 may be such that more than 50% of the beads within the spiral of beads 1104 is included within the X-ray beam 106. This arrangement may allow the PCCT system to image a target that includes a plurality of beads.

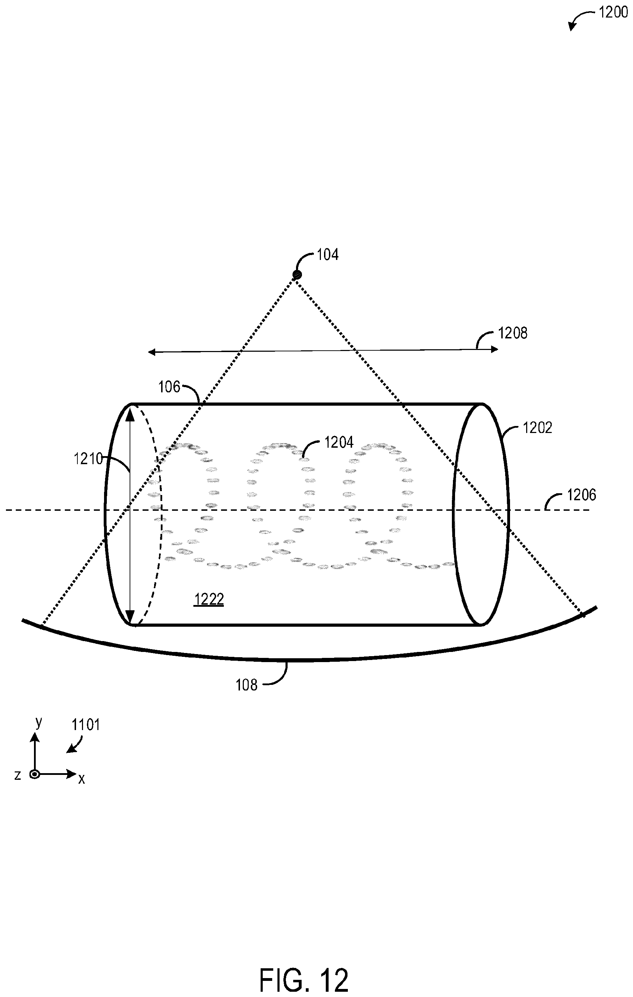

FIG. 12 depicts a schematic diagram 1200 of a first embodiment of a cylindrical phantom 1202 that includes beads. The first embodiment of the cylindrical phantom 1202 may be a cylindrical with a length 1208 along the x-axis. In one example the length 1208 may be 40 cm. The first embodiment of a cylindrical phantom 1202 may be positioned between the X-ray source 104 and the detector array 108 such that the X-ray beam 106 from the X-ray source 104 may be attenuated by the first embodiment of a cylindrical phantom 1202 and strike the detector array 108.

The first embodiment of the cylindrical phantom 1202 may have a two circular faces in that lie in the y-z plane. The circular faces may have a diameter 1210 that may be less than the length 1208. A midline 1206 may run through the center of circular faces of the first embodiment of the cylindrical phantom 1202 along the x-axis. As described above with reference to FIG. 11, the cylindrical phantom 1202 may comprise a base material 1222, which may include any of the aforementioned base materials or other clinically relevant materials, e.g., PVC, PE, metal, air, and so on. The cylindrical phantom 1202 may include a spiral of beads 1204 that may mimic clinical findings, such as the beads described in more detail with respect to FIGS. 6A-6C. The spiral of beads 1204 may form a circular spiral centered around the midline 1206. The radius of the spiral, number of beads within the spiral, size of bead and material of bead may or may not be held constant within the first embodiment of a cylindrical phantom 1202. The length 1208 of the first embodiment of a cylindrical phantom 1202 and the position of the first embodiment of a cylindrical phantom 1202 may be such that more than 50% of the beads within the spiral of beads 1204 are included within the X-ray beam 106. This arrangement may allow the PCCT system to image a target that includes a plurality of beads.

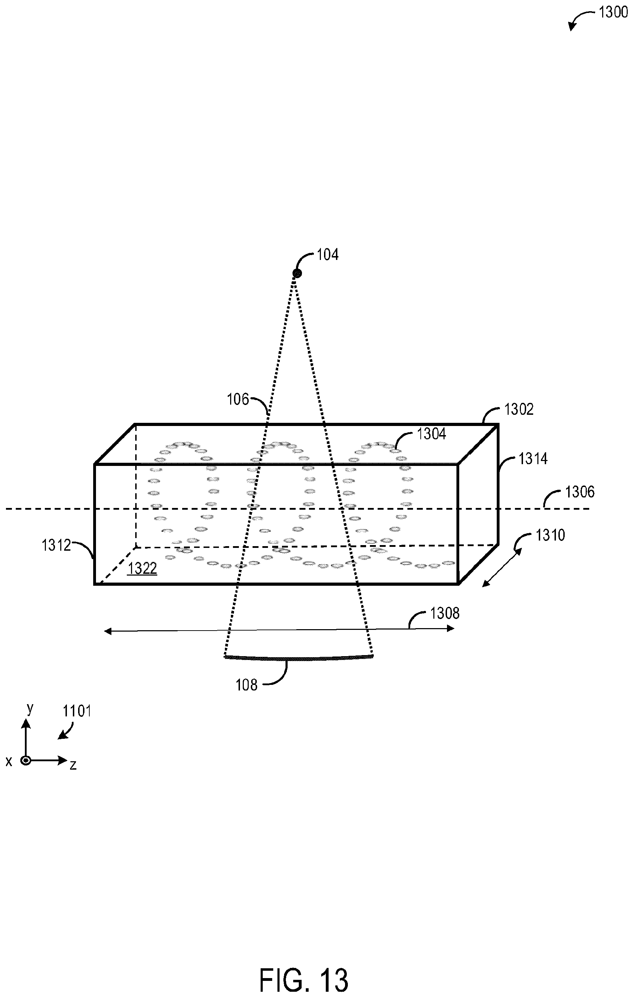

FIG. 13 depicts a schematic diagram 1300 of a second embodiment of a rectangular prism phantom 1302 that includes beads. The second embodiment of the rectangular prism phantom 1302 may be a rectangular prism with a length 1308 along the z-axis. In one example the length 1308 may be 40 cm. The second embodiment of a rectangular prism phantom 1302 may be positioned between the X-ray source 104 and the detector array 108 such that the X-ray beam 106 from the X-ray source 104 may be attenuated by the second embodiment of a rectangular prism phantom 1302 and strike the detector array 108.

The second embodiment of the rectangular prism phantom 1302 may have a rectangular front face 1312 and a rectangular back face 1314 that may lie in parallel y-x planes, separated by the length 1108. Rectangular faces may couple the front face 1312 and the back face 1314 along each of their respective sides to form a rectangular prism. The front face 1312 and the back face 1314 may have a width 1310 along the x-axis of between 20 cm and 40 cm. In one example, the front face 1312 and the back face 1314 may be squares. A midline 1306 may run through the center of the front face 1312 and the back face 1314 along the z-axis. As described above with reference to FIG. 11, the rectangular prism phantom 1302 may comprise a base material 1322, which may include any of the aforementioned base materials or other clinically relevant materials, e.g., PVC, PE, metal, air, and so on. The rectangular prism phantom 1302 may include a spiral of beads 1304 that may mimic clinical findings, such as the beads described in more detail with respect to FIGS. 6A-6C. The spiral of beads 1304 may form a circular spiral centered around the midline 1306. The radius of the spiral, number of beads within the spiral, size of bead and material of bead may or may not be held constant within the second embodiment of a rectangular prism phantom 1302. The length 1308 of the first embodiment of a rectangular prism phantom 1302 and the position of the first embodiment of a rectangular prism phantom 1302 may be such that less than 50% of the beads within the spiral of beads 1304 are included within the X-ray beam 106. This arrangement may allow the PCCT system to image a target that includes a select portion of the beads within the spiral of beads 1304.

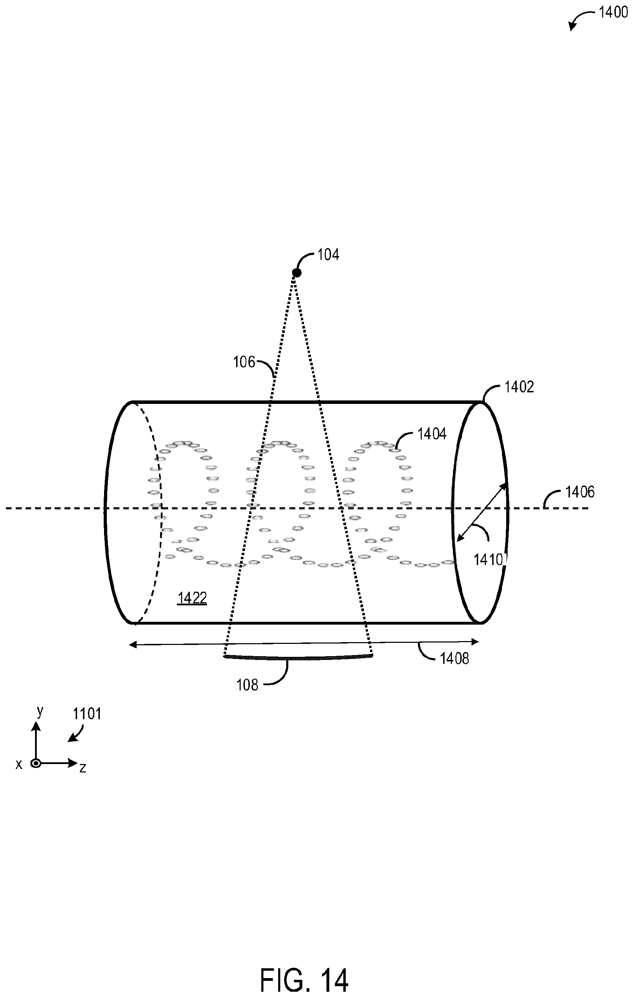

FIG. 14 depicts a schematic diagram 1400 of a second embodiment of a cylindrical phantom 1402 that includes beads. The second embodiment of the cylindrical phantom 1402 may be a cylindrical with a length 1408 along the z-axis. In one example the length 1408 may be 40 cm. The second embodiment of a cylindrical phantom 1402 may be positioned between the X-ray source 104 and the detector array 108 such that the X-ray beam 106 from the X-ray source 104 may be attenuated by the second embodiment of a cylindrical phantom 1402 and strike the detector array 108.

The second embodiment of the cylindrical phantom 1402 may have a two circular faces in that lie in the y-x plane. The circular faces may have a diameter 1410 that may be less than the length 1408. A midline 1406 may run through the center of circular faces of the first embodiment of the cylindrical phantom 1402 along the z-axis. As described above with reference to FIG. 11, the cylindrical phantom 1402 may comprise a base material 1422, which may include any of the aforementioned base materials or other clinically relevant materials, e.g., PVC, PE, metal, air, and so on. The cylindrical phantom 1402 may include a spiral of beads 1404 that may mimic clinical findings, such as the beads described in more detail with respect to FIGS. 6A-6C. The spiral of beads 1404 may form a circular spiral centered around the midline 1406. The radius of the spiral, number of beads within the spiral, size of bead and material of bead may or may not be held constant within the first embodiment of a cylindrical phantom 1402. The length 1408 of the second embodiment of a cylindrical phantom 1402 and the position of the second embodiment of a cylindrical phantom 1402 may be such that less than 50% of the beads within the spiral of beads 1404 are included within the X-ray beam 106. This arrangement may allow the PCCT system to image a target that includes a select portion of the beads within the spiral of beads 1404.

Thus, the systems and methods disclosed herein provide an approach for calibrating a photon counting computing tomography system using a non-uniform phantom. The non-uniform phantom, by incorporating clinically relevant object sizes and contrasts, in combination with the disclosed calibration methods, captures PC detector charge sharing, and detector scatter or crosstalk behavior using calibration scans. The PC detector response is used to adjust one or more detector response models, such as a model-based crosstalk correction algorithm, and the one or more detector response models are applied as one or more corrections on the patient data before final images are generated. Material density accuracy, spatial resolution, low contrast detectability may be improved with this approach.

The technical effect of the disclosed systems and methods is increased accuracy over other methods of calibrating a photon counting detector, resulting in the production of higher quality of CT images.

Though a photon counting computed tomography (PCCT) system is described by way of example, it should be understood that the present techniques may also be useful when applied to other X-ray imaging modalities having photon counting detectors, such as X-ray angiography systems, X-ray tomosynthesis systems, X-ray mammography systems, X-ray fluoroscopy systems, X-ray interventional systems, X-ray C-arm systems, etc. The present discussion of a PCCT imaging modality is provided merely as an example of one suitable imaging modality.

The disclosure also provides support for a method for a photon counting computed tomography (PCCT) system, the method comprising: during a calibration of the PCCT system: generating a detector response prediction for one or more known composition features of a non-uniform phantom, performing a calibration scan of the non-uniform phantom at a plurality of positions between an X-ray source and a detector of the PCCT system, measuring an actual detector response at each of the plurality of positions, generating a correction factor based on the detector response prediction and the actual detector response at each of the plurality of positions, and adjusting a calibration algorithm based on the correction factor. In a first example of the method, the method further comprises: applying the calibration algorithm to perform a scan of a subject. In a second example of the method, optionally including the first example, the non-uniform phantom comprises a base material and the one or more known composition features are arranged in the base material, the one or more known composition features comprising a different material than the base material. In a third example of the method, optionally including one or both of the first and second examples, generating the detector response prediction comprises calculating a contrast prediction based on a material composition of the one or more known composition features relative to the material composition of the base material. In a fourth example of the method, optionally including one or more or each of the first through third examples, the calibration algorithm is a model-based detector crosstalk and charge sharing correction algorithm. In a fifth example of the method, optionally including one or more or each of the first through fourth examples, measuring the actual detector response comprises measuring a level of focal spot blurring at each of the plurality of positions. In a sixth example of the method, optionally including one or more or each of the first through fifth examples, the calibration algorithm is one or more of a spectral correction algorithm and a patient/scanner scatter correction algorithm. In a seventh example of the method, optionally including one or more or each of the first through sixth examples, the one or more known composition features comprise one or more objects of a known height and a known diameter arranged at a known depth within a base material, one or both of a size and a material of clinical relevance, and one or more objects of a known size and a known contrast. In a eighth example of the method, optionally including one or more or each of the first through seventh examples, the one or more known composition features comprise a plurality of slots filled with one or more materials comprising one or more of a pure, composite, doped, coated, organic, inorganic, solid, or fluid material. In a ninth example of the method, optionally including one or more or each of the first through eighth examples, the non-uniform phantom comprises a cylindrical or elliptical base material and the one or more known composition features comprise micro or sub-micro scale beads of various materials and densities.

The disclosure also provides support for a non-uniform phantom for a photon counting computed tomography (PCCT) system, comprising: a base material, and one or more known composition features embedded in the base material, the one or more known composition features comprising a different material than the base material, wherein the one or more known composition features comprise clinically relevant object sizes and contrasts, and wherein the base material and the one or more known composition features are selected based on one or more of a calibration method, a clinical purpose, and a research purpose. In a first example of the method, the calibration method comprises correcting for one or both of charge sharing and detector crosstalk. In a second example of the method, optionally including the first example, the one or more known composition features comprise one or more of a hole, slot, recess, or bore in the base material. In a third example of the method, optionally including one or both of the first and second examples, the hole, slot, recess, or bore comprise a filler material. In a fourth example of the method, optionally including one or more or each of the first through third examples, the hole, slot, recess, or bore and the filler material comprise a standardized size, and wherein the filler material is interchangeable to assemble different material combinations. In a fifth example of the method, optionally including one or more or each of the first through fourth examples, the base material is air. In a sixth example of the method, optionally including one or more or each of the first through fifth examples, the non-uniform phantom comprises a cylindrical or elliptical base material and the one or more known composition features comprise micro or sub-micro scale beads of various materials and densities.

The disclosure also provides support for a photon counting computed tomography (PCCT) system, comprising: an X-ray source that emits a beam of X-rays toward a subject to be imaged, a photon counting detector that receives the beam of X-rays attenuated by the subject, a non-uniform phantom, and a data acquisition system (DaS) operably connected to the photon counting detector and configured to: during a calibration of the PCCT system, generate a detector response prediction for one or more known composition features of the non-uniform phantom, perform a calibration scan of the non-uniform phantom at a plurality of positions between the X-ray source and the photon counting detector of the PCCT system, measure an actual detector response at each of the plurality of positions, generate a correction factor based on the detector response prediction and the actual detector response at each of the plurality of positions, and adjust one or more calibration algorithms based on the correction factor. In a first example of the system, the non-uniform phantom comprises a base material and the one or more known composition features are arranged in the base material, the one or more known composition features comprising a different material than the base material, and wherein the detector response prediction comprises a contrast prediction based on a material composition of the one or more known composition features relative to the material composition of the base material. In a second example of the system, optionally including the first example, the one or more calibration algorithms are one or more of a model-based detector crosstalk and charge sharing correction algorithm, a spectral correction algorithm, and a patient/scanner scatter correction algorithm.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative only and should not be construed to be limiting in any manner.

## Claims

1. A method (800, 900, 1000) for a photon counting computed tomography (PCCT) system (100), the method comprising:
during a calibration of the PCCT system (100):
generating a detector response prediction (901) for one or more known composition features of a non-uniform phantom (302, 602);
performing a calibration scan (904) of the non-uniform phantom (302, 602) at a plurality of positions (902) between an X-ray source (104) and a detector of the PCCT system (100);
measuring an actual detector response (906) at each of the plurality of positions;
generating a correction factor (912) based on the detector response prediction and the actual detector response at each of the plurality of positions; and
adjusting a calibration algorithm based on the correction factor (1000).

2. The method (800, 900, 1000) of claim 1, further comprising applying the calibration algorithm to perform a scan of a subject (204, 112).

3. The method (800, 900, 1000) of claim 1, wherein the non-uniform phantom (302, 602) comprises a base material and the one or more known composition features (304) are arranged in the base material, the one or more known composition features comprising a different material than the base material.

4. The method (800, 900, 1000) of claim 3, wherein generating the detector response prediction comprises calculating a contrast prediction based on a material composition of the one or more known composition features relative to the material composition of the base material (901).

5. The method (800, 900, 1000) of claim 1, wherein the calibration algorithm is a model-based detector crosstalk and charge sharing correction algorithm (912).

6. The method (800, 900, 1000) of claim 1, wherein measuring the actual detector response comprises measuring a level of focal spot blurring at each of the plurality of positions (904).

7. The method (800, 900, 1000) of claim 1, wherein the calibration algorithm is one or more of a spectral correction algorithm and a patient/scanner scatter correction algorithm (912).

8. The method (800, 900, 1000) of claim 1, wherein the one or more known composition features comprise one or more objects of a known height and a known diameter (304) arranged at a known depth within a base material (302), one or both of a size and a material of clinical relevance, and one or more objects of a known size and a known contrast.

9. The method (800, 900, 1000) of claim 1, wherein the one or more known composition features comprise a plurality of slots (304) filled with one or more materials comprising one or more of a pure, composite, doped, coated, organic, inorganic, solid, or fluid material.

10. The method (800, 900, 1000) of claim 1, wherein the non-uniform phantom (602) comprises a cylindrical or elliptical base material (622) and the one or more known composition features comprise micro or sub-micro scale beads (603) of various materials and densities.

11. A non-uniform phantom (302, 602) for a photon counting computed tomography (PCCT) system, comprising:
a base material (302, 622); and
one or more known composition features (304) embedded in the base material (302), the one or more known composition features comprising a different material than the base material,
wherein the one or more known composition features comprise clinically relevant object sizes and contrasts, and
wherein the base material (302, 622) and the one or more known composition features are selected based on one or more of a calibration method, a clinical purpose, and a research purpose (700).

12. The non-uniform phantom (302, 602) of claim 11, wherein the calibration method (900, 800) comprises correcting for one or both of charge sharing and detector crosstalk.

13. The non-uniform phantom (302, 602) of claim 11, wherein the one or more known composition features comprise one or more of a hole, slot, recess, or bore (304) in the base material.

14. The non-uniform phantom (302, 602) of claim 13, wherein the hole, slot, recess, or bore comprise a filler material (304).

15. The non-uniform phantom (302, 602) of claim 14, wherein the hole, slot, recess, or bore (304) and the filler material comprise a standardized size, and wherein the filler material
